# EUROPÄISCHE PATENTSCHRIFT

(11) **EP 2 811 961 B1**
(45) Veröffentlichungstag und Bekanntmachung des Hinweises auf die Patenterteilung: **02.08.2017**
(21) Anmeldenummer: 13707565.1
(22) Anmeldetag: 07.02.2013
(51) Int. Cl.: A61G 13/10, A61G 13/12, A61G 1/056

(54) **VORRICHTUNG ZUM MANUELLEN ENTRIEGELN EINES MIT EINER LAST BEAUFSCHLAGBAREN HALTEMECHANISMUS**
DEVICE FOR MANUALLY UNLOCKING A HOLDING MECHANISM THAT CAN BE LOADED WITH A LOAD
DISPOSITIF POUR DÉVERROUILLER MANUELLEMENT UN MÉCANISME DE RETENUE POUVANT ÊTRE SOUMIS À UNE CHARGE

(30) Priorität: 07.02.2012 DE 102012100970
(43) Veröffentlichungstag der Anmeldung: 17.12.2014
(73) Patentinhaber: Maquet GmbH, 76437 Rastatt (DE)
(72) Erfinder: WYSLUCHA, Ulrich, 76356 Weingarten (DE); SINGER, Markus, 73207 Plochingen (DE); GANTKE, Reinhard, 72663 Großbettlingen (DE); HOPFENGART, Hans-Jörg, 73773 Aichwald (DE)
(74) Vertreter: Zacco GmbH
(86) Internationale Anmeldenummer: PCT/EP2013/052408
(87) Internationale Veröffentlichungsnummer: WO 2013/117632

(56) Entgegenhaltungen:
- WO-A2-2005/007053
- US-A- 4 881 728
- US-A- 5 537 700
- US-A1- 2006 117 485

## Beschreibung

Die Erfindung betrifft eine Vorrichtung zum manuellen Entriegeln eines mit einer Last beaufschlagbaren Haltearms, umfassend ein Bedienteil, das zum Entriegeln des Haltearms mit einer Betätigungskraft, die mit zunehmender Last größer wird, manuell zu betätigen ist, einen Umsetzmechanismus, der ein mit dem Bedienteil gekoppeltes Kraftübertragungselement aufweist, und einen mit dem Kraftübertragungselement gekoppelten Auslöser, der zum Entriegeln des Haltearms durch die Betätigungskraft, die das Kraftübertragungselement auf den Auslöser überträgt, aus einer Verriegelungsstellung, in welcher der Auslöser von dem Haltearm wirkungsmäßig entkoppelt ist, in eine Entriegelungsstellung bewegbar ist, in welcher der Auslöser mit dem Haltearm zu dessen Entriegelung wirkungsmäßig gekoppelt ist.

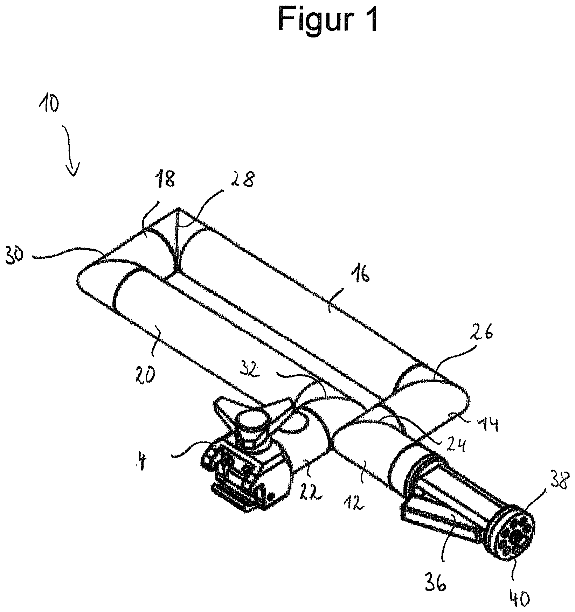

In der Medizintechnik kommt seit kurzem ein mechanisches Assistenzsystem z.B. zur Armlagerung bei Operationen an der Schulter oder am Oberarm eines Patienten zum Einsatz. Dieses Assistenzsystem ist im Wesentlichen aus einem Haltearm gebildet, der rein schematisch in Figur 1 dargestellt ist.

Der in Figur 1 mit 10 bezeichnete Haltearm weist mehrere starre Halteglieder 12, 14, 16, 18, 20 und 22 auf, die über Gelenke 24, 26, 28, 30 und 32 miteinander gekoppelt sind. An einem Ende des Haltearms 10 ist eine Befestigungsvorrichtung 34 angeordnet, die dazu dient, den Haltearm 10 an einer Gleitschiene eines OP-Tisches anzubringen. An dem anderen Ende des Haltearms 10 befindet sich ein Handgriff 36, der von dem Anwender manuell betätigt werden kann, um den Haltearm 10 in nachfolgend beschriebener Weise zu entriegeln. An dem Haltegriff 36 ist eine mit mehreren Rastöffnungen 38 versehene Halterung 40 angebracht, an der eine nicht gezeigte Auflage z.B. zur Lagerung des Patientenarms angebracht werden kann.

Wie vorstehend erwähnt, dient der Handgriff 36 der Entriegelung des Haltearms 10, insbesondere der die einzelnen Halteglieder 12 bis 22 miteinander koppelnden Gelenke 24 bis 32. Hierzu enthält der Haltegriff 36 einen in Figur 1 nicht gezeigten mechanischen Auslöser, der beim Zusammendrücken des Handgriffs 36 ein durch den Haltearm 10 geführtes, in Figur 1 nicht gezeigtes Entriegelungsgestänge bestätigt. Dabei wird die auf den Handgriff 36 ausgeübte Betätigungskraft über einen Umsetzmechanismus, der sich innerhalb des Handgriffs 36 befindet, auf den Auslöser übertragen. Wirkt der Auslöser durch Betätigen des Handgriffs 36 auf das Entriegelungsgestänge ein, so sorgt letzteres dafür, dass die einzelnen Gelenke 24 bis 32 entriegelt werden. Jedes der Gelenke 24 bis 32 ist so ausgebildet, dass es im entriegelten Zustand eine Relativbewegung der beiden Halteglieder ermöglicht, die durch dieses Gelenk miteinander gekoppelt sind.

Wird keine Betätigungskraft auf den Handgriff 36 ausgeübt, so sind also die Halteglieder 12 bis 22 des Haltearms 10 über die Gelenke 24 bis 32 starr miteinander gekoppelt. In diesem Zustand bildet der Haltearm 10 eine starre Einheit, die geeignet ist, den Patientenarm stabil zu lagern. Soll die Lage des Patientenarms im Raum verändert werden, so drückt der Anwender den Handgriff 36 zusammen, wodurch die Entriegelungsmechanik über den mechanischen Auslöser betätigt wird. Durch die Betätigung des Handgriffs 36 werden so die über die Gelenke 24 bis 32 miteinander gekoppelten Halteglieder 12 bis 22 gegeneinander beweglich, so dass der Anwender den Haltearm 10 nach Wunsch ausrichten kann. Lässt anschließend der Anwender den Haltegriff 36 wieder los, so werden die Gelenke 24 bis 32 wieder verriegelt, und der Haltearm 10 wird in seiner veränderten Ausrichtung fixiert.

Die Betätigungskraft, die der Anwender auf den Handgriff 36 zur Entriegelung des Haltearms 10 ausüben muss, ist abhängig von der Last, mit der der Haltearm 10 beaufschlagt ist. Diese Last setzt sich aus dem Eigengewicht des Haltearms 10 und dem Gewicht des gelagerten Patientenarms zusammen. Je schwerer der Patientenarm ist, desto größer muss demnach die Betätigungskraft sein, die der Anwender zur Entriegelung des Haltearms 10 auf den Handgriff 36 ausüben muss.

Betätigt der Anwender den Handgriff 36 mit einer ausreichend großen Betätigungskraft, so wird der Haltearm 10 schlagartig entriegelt. Dies bedeutet, dass der Haltearm 10 augenblicklich der Last, d.h. im Wesentlichen dem Gewicht des Patientenarms nachgibt. Dieses Nachgeben des Haltearms 10 ist weniger problematisch, solange das auf dem Haltearm 10 gelagerte Gewicht relativ gering ist. In diesem Fall kann der Anwender einem plötzlichen Absacken des Haltearms 10 dadurch entgegenwirken, dass er mit seiner Hand, die den Handgriff 26 hält, auf eine dem Gewicht des Patientenarms entgegengesetzte Gegenkraft ausübt, also im Regelfall, in dem die Last nach unten wirkt, den Haltegriff 36 etwas nach oben drückt.

Ist jedoch das Gewicht des gelagerten Patientenarms vergleichsweise groß, so muss der Anwender nach dem Entriegeln des Haltearms 10 eine entsprechend große Gegenkraft ausüben, um den Haltearm 10 zu stabilisieren. Da die Entriegelung des Haltearms 10 schlagartig eintritt, muss der Anwender schnell reagieren, was die Handhabung des Haltearms 10 erschwert. Nun kann der Anwender ein schlagartiges Absacken des Haltearms 10 von vorneherein dadurch vermeiden, dass er, während er mit einer Hand den Handgriff 36 betätigt, mit seiner anderen Hand den Haltearm 10 von unten abstützt. Dies erleichtert im Übrigen auch die Entriegelung des Haltearms 10. Jedoch kommt es in der Praxis häufig vor, dass der Anwender diese Sicherungsmaßnahme zur Unterstützung des Haltearms 10 vergisst und so davon überrascht wird, dass der Haltearm 10 beim Entriegeln plötzlich seine Stabilität verliert und nach unten absackt. Dies erschwert die Handhabung des Haltearms 10.

WO 2005/007053 A2 offenbart eine Patiententransportvorrichtung mit einem Unterwagen und einer Patiententragestruktur, die auf dem Unterwagen sitzt. Um die Höhe der Patiententragestruktur einstellen zu können, ist an dem Unterwagen ein Handgriff zum Steuern eines Druckluftventils vorgesehen, das den Luftstrom in zwei Pneumatikzylinder steuert. Der Handgriff ist mit einer Schubstange gekoppelt, die wiederum an einer Arretierleiste angreift. Über die Schubstange lässt sich die Arretierleiste um ihre Längsachse aus einer vertikalen neutralen Stellung in zwei entgegengesetzte Entriegelungsstellungen drehen. Die Arretierleiste ist über zwei Federn in die Neutralstellung vorgespannt. Der Handgriff hat somit sowohl die Funktion, die Arretierleiste zu entriegeln, als auch die Funktion, den Luftstrom in die Pneumatikzylinder zum Anheben oder Absenken der Patiententragestruktur zu steuern.

Zum Stand der Technik wird ferner auf die US 5 537 700 A verwiesen, die eine Tragevorrichtung mit einem Lagermechanismus offenbart, der eine Patiententrage vertikal verstellbar auf einer mit Rädern versehenen Basis hält. Die Beine des Lagermechanismus sind jeweils längenverstellbar. Über eine Verriegelungsanordnung kann die Patiententrage in einer gewünschten vertikalen Position verriegelt werden. Die Verriegelungsanordnung kann nur dann gelöst werden, wenn die Patiententrage manuell angehoben wird.

US 2006/117485 A1 zeigt eine Fußstütze für ein Entbindungsbett mit einem Entriegelungsmechanismus, bei dem ein Verriegelungsstift mit einer Feder vorgespannt ist und in entsprechende Öffnungen eingreift.

Aufgabe der Erfindung ist es, eine für einen Haltearm bestimmte, manuell betätigbare Entriegelungsvorrichtung eingangs genannter Art so weiterzubilden, dass deren Handhabung einfacher und sicherer als bisher wird.

Die Erfindung löst diese Aufgabe durch die Vorrichtung nach Anspruch 1. Der Umsetzmechanismus weist einen elastisch verformbaren Kraftbegrenzer auf, über den das Kraftübertragungselement mit dem Auslöser gekoppelt ist und der durch seine elastische Verformung die Übertragung der Betätigungskraft auf den Auslöser verhindert, wenn die Betätigungskraft eine vorbestimmte Kraft übersteigt.

Die Erfindung macht sich den Umstand zunutze, dass die Betätigungskraft, die zum Entriegeln des Haltearms manuell auf den Bedienteil auszuüben ist, von der zu lagernden Last abhängig ist, insbesondere mit zunehmender Last größer wird. Dies bedeutet, dass die zum Entriegeln des Haltearms aufzubringende Kraft als ein Maß für die Last angesehen werden kann, mit der der Haltearm beaufschlagt ist. Der Umstand, dass die Betätigungskraft mit zunehmender Last größer wird, beinhaltet auch einen Sicherheitsaspekt dahingehend, dass ein versehentliches Entriegeln bei großer Last vermieden wird.

Die Erfindung sieht einen elastisch verformbaren Kraftbegrenzer vor, der eine Übertragung der manuell auf das Bedienteil ausgeübten Betätigungskraft auf den Auslöser nur dann gestattet, wenn die Betätigungskraft nicht größer als eine vorbestimmte Kraft ist. Da die Betätigungskraft, wie vorstehend erläutert, ein Maß für die auf de Haltearm ruhende Last ist, verhindert der Kraftbegrenzer eine Übertragung der Betätigungskraft auf den Auslöser und damit eine Entriegelung des mit der Last beaufschlagten Haltearm, wenn diese Last einen vorbestimmten Wert übersteigt. Dies bedeutet umgekehrt, dass eine Entriegelung des Haltearms allein durch Betätigen des Bedienteils erfindungsgemäß nur dann möglich ist, wenn die Last nicht größer als dieser vorbestimmte Wert ist.

Betätigt der Anwender das Bedienteil und stellt er dabei fest, dass ihm dadurch ein Entriegeln des Haltearms nicht möglich ist, so wird dem Anwender bewusst, dass die auf dem Haltearm ruhende Last größer als der vorbestimmte Wert ist. Nachdem der Anwender dies erkannt hat, kann er den Haltearm beispielsweise, während er mit einer Hand weiterhin die Betätigungskraft auf das Bedienteil ausübt, mit derselben oder seiner anderen Hand den Haltemechanismus unterstützen, um der auf dem Haltearm ruhenden Last entgegenzuwirken und damit den Haltearm zu entlasten. Ist der Haltearm auf diese Weise ausreichend entlastet, so wird dem Anwender möglich, durch Ausüben der Betätigungskraft auf das Bedienteil den Haltearm zu entriegeln. Dadurch wird zuverlässig vermieden, dass der Anwender von einem schlagartigen Übergang des Haltearms aus einem lasthaltenden Zustand in einem lastnachgebenden Zustand überrascht wird. Außerdem wird durch die Begrenzung der wirksamen Betätigungskraft eine Beschädigung der die Entriegelungsvorrichtung und/oder den Haltearm bildenden Teile in Folge einer übermäßigen Krafteinwirkung verhindert. Schließlich ist durch die Begrenzung der Betätigungskraft eine gute Ergonomie der Griffbetätigung gewährleistet.

In einer bevorzugten Ausgestaltung ist der Kraftbegrenzer eine Feder, deren Rückstellkraft so bemessen, dass die Feder ihre ursprüngliche Form beibehält, wenn die Betätigungskraft kleiner oder gleich der vorbestimmten Kraft ist, und dass die Feder sich elastisch verformt, wenn die Betätigungskraft die vorbestimmte Kraft übersteigt. Ist die Betätigungskraft hinreichend klein, so wirkt die zwischen das Kraftübertragungselement und den Auslöser geschaltete Feder wie ein im Wesentlichen starres Element, so dass die auf das Bedienteil ausgeübte Betätigungskraft über die Feder weitgehend ungeschwächt auf den Auslöser wirkt und der Haltearm entriegelt wird. Wird dagegen die auf das Bedienteil ausgeübte Betätigungskraft so groß, dass der Anwender den Haltearm vor seiner Entriegelung entlasten sollte, um nicht von der schlagartig einsetzenden Instabilität des Haltearms überrascht zu werden, so absorbiert die Feder in Folge ihrer elastischen Verformung gleichsam die ausgeübte Betätigungskraft, wodurch letztere nicht mehr auf den Auslöser wirkt und der Haltearm verriegelt bleibt. Erst mit Entlastung des Haltearms wird die auf das Betätigungsteil auszuübende Betätigungskraft wieder so klein, dass diese keine elastische Verformung der Feder bewirkt und die Betätigungskraft auf den Auslöser übertragen wird.

Vorzugsweise ist die Feder eine Druckfeder, die zwischen dem Kraftübertragungselment und dem Auslöser zusammengedrückt wird, wenn die auf das Bedienteil ausgeübte Betätigungskraft größer als die vorbestimmte Kraft ist.

In einer vorteilhaften Ausführung umfasst der Umsetzmechanismus eine an dem Bedienteil ausgebildete erste Zahnung und eine an dem Kraftübertragungselement ausgebildete zweite Zahnung, die in Eingriff mit der ersten Zahnung steht. Ein solches aus zwei Zahnungen gebildetes Getriebe ist besonders geeignet, eine Betätigung des Bedienteils beispielsweise in eine Linearbewegung des Kraftübertragungselementes und damit in eine entsprechende Bewegung des über den Kraftbegrenzer mit dem Kraftübertragungselement gekoppelten Auslösers umzusetzen (sofern die Betätigungskraft eine vorbestimmte Kraft nicht übersteigt).

In einer alternativen Ausführungsform umfasst der Umsetzmechanismus eine an dem Bedienteil ausgebildete, bewegliche Andruckfläche und einen Kniehebel mit einem längeren ersten Schenkel und einem kürzeren zweiten Schenkel, wobei der längere erste Schenkel des Kniehebels an einem Ende in einem innerhalb der Entriegelungsvorrichtung ortsfesten ersten Drehpunkt gelagert ist, der kürzere zweite Schenkel des Kniehebels an einem Ende in einem zweiten Drehpunkt, der relativ zu dem beweglich geführten Kraftübertragungselement ortsfest ist, drehbar gelagert ist, die beiden Schenkel jeweils an ihrem anderen Ende in einem gemeinsamen dritten Drehpunkt gelagert sind, und die Andruckfläche beim manuellen Betätigen des Bedienteils im Bereich des dritten Drehpunkts auf den Kniehebel drückt. Durch die Verwendung eines Kniehebels lässt sich eine verzögerte Übersetzung der Betätigungskraft bei gleichbleibender Betätigungsgeschwindigkeit erzielen. Dies bedeutet, dass bei gleichbleibender Betätigungsgeschwindigkeit die Hubgeschwindigkeit, mit der das Kraftübertragungselement bewegt wird, abnimmt, während die durch das Kraftübertragungselement ausgeübte Kraft zunimmt. Die von dem Anwender ausgeübte Betätigungskraft kommt so verzögert zur Wirkung, so dass sich der Anwender bei Betätigen des Bedienteils besser auf die bevorstehende Entriegelung des Haltearms einstellen kann. Außerdem wird so eine größere Kraftübersetzung möglich.

Vorzugsweise weist das Kraftübertragungselement einen hohlzylindrischen Abschnitt auf, in dem der Kraftbegrenzer gelagert ist. Dies ermöglicht einen besonders kompakten Aufbau der Entriegelungsvorrichtung. Der Auslöser umfasst beispielsweise eine Auslösestange, die in Richtung ihrer Längsachse linear geführt ist. Die Auslösestange kann in diesem Fall zumindest teilweise innerhalb des länglich geformten Kraftbegrenzers geführt sein. Ist der Kraftbegrenzer z.B. eine Schraubenfeder, so ragt in dieser Ausführung ein Teil der Auslösestange in das Innere der Schraubenfeder. Auch dies begünstigt einen besonders kompakten Aufbau der Entriegelungsvorrichtung.

Vorzugsweise ist ein Anschlag vorgesehen, der die elastische Verformung des Kraftbegrenzers begrenzt. Ist der Kraftbegrenzer beispielsweise eine Druckfeder, so gewährleistet der Anschlag, dass die Druckfeder nur so stark zusammengedrückt wird, dass der dadurch erzeugte Federweg noch innerhalb eines durch eine vordefinierte Federkennlinie festgelegten Arbeitsbereichs liegt. Dadurch ist sichergestellt, dass die Feder ihre Funktion, nämlich die Übertragung der auf das Bedienteil ausgeübten Betätigungskraft auf den Auslöser in Abhängigkeit der Größe dieser Kraft entweder zu ermöglichen oder zu verhindern, über lange Zeit zuverlässig erfüllen kann.

Der vorstehend genannte Anschlag ist beispielsweise ein mit dem Kraftübertragungselement in Anlage kommende Anschlagfläche. In dieser Ausführungsform begrenzt der Anschlag den Hub des Kraftübertragungselementes. Es ist jedoch ebenso möglich, den Anschlag beispielsweise dem Bedienteil zuzuordnen, um dessen Betätigungsweg einzuschränken.

Nach einem weiteren Aspekt der Erfindung ist ein mit einer Last beaufschlagbarer Haltearm vorgesehen, der eine Entriegelungsvorrichtung vorstehend beschriebener Art umfasst.

Die Erfindung wird im Folgenden anhand der Figuren näher erläutert. Darin zeigen:
- Fig. 1: einen herkömmlichen Haltearm zum Lagern eines Patientenarms;
- Fig. 2: einen Längsschnitt durch eine auf den Haltearm nach Figur 1 anwendbare Entriegelungsvorrichtung gemäß erstem Ausführungsbeispiel in einem Zustand, in dem ein Handgriff der Entriegelungsvorrichtung nicht betätigt und der Haltearm verriegelt ist;
- Fig. 3: einen Längsschnitt durch die Entriegelungsvorrichtung nach Figur 2 in einem Zustand, in dem der Handgriff betätigt und dadurch der Haltearm entriegelt ist;
- Fig. 4: einen Längsschnitt durch die Entriegelungsvorrichtung nach Figur 2 in einem Zustand, in dem der Handgriff betätigt und der Haltearm trotzdem verriegelt bleibt;
- Fig. 5: einen Längsschnitt durch eine auf den Haltearm nach Figur 1 anwendbare Entriegelungsvorrichtung gemäß zweitem Ausführungsbeispiel in einem Zustand, in dem der Handgriff der Entriegelungsvorrichtung nicht betätigt und der Haltearm verriegelt ist;
- Fig. 6: einen Längsschnitt durch die Entriegelungsvorrichtung nach Figur 5 in einem Zustand, in dem der Handgriff betätigt und der Haltearm entriegelt ist; und
- Fig. 7: einen Längsschnitt durch die Entriegelungsvorrichtung nach Figur 5 in einem Zustand, in dem der Handgriff betätigt und der Haltearm trotzdem verriegelt bleibt.

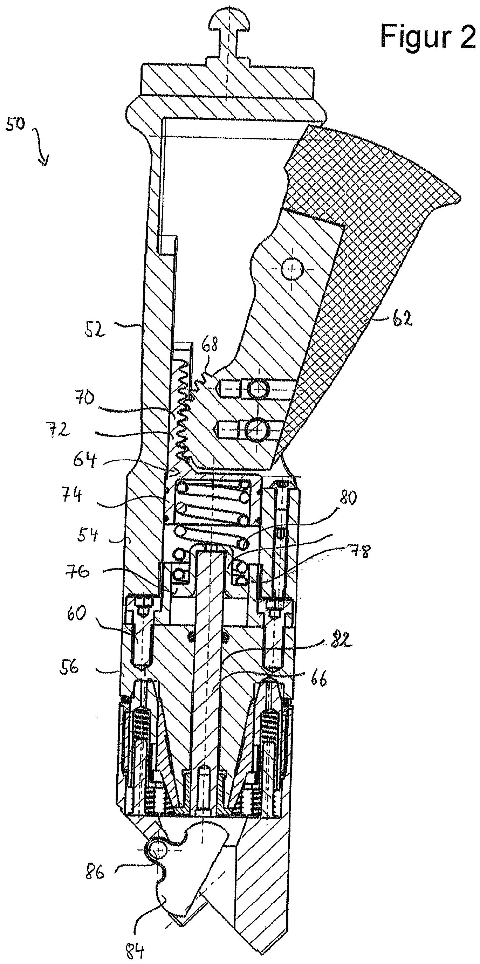

Figur 2 zeigt eine Entriegelungsvorrichtung 50 als erstes Ausführungsbeispiel in einem Längsschnitt. Die Entriegelungsvorrichtung 50 ist geeignet, den in Figur 1 gezeigten Haltearm 10 in nachfolgend beschriebener Weise zu entriegeln.

Die Entriegelungsvorrichtung 50 hat ein längliches Gehäuse 52, das aus zwei Gehäuseteilen 54 und 56 gebildet ist, die durch Schraubverbindungen 58 und 60 miteinander gekoppelt sind. Die Entriegelungsvorrichtung 50 weist ferner einen von einem Anwender manuell betätigbaren Handgriff 62, einen in Figur 2 allgemein mit 64 bezeichneten Umsetzmechanismus und eine entlang der Gehäuselängsachse beweglich geführte Auslösestange 66 auf. Der Umsetzmechanismus 64 ist in dem ersten Ausführungsbeispiel durch ein Getriebe gebildet, das einen an dem Handgriff 62 ausgebildeten Zahnradabschnitt 68 und eine mit dem Zahnradabschnitt 68 in Eingriff stehende Zahnschiene 70 umfasst. Die Zahnschiene 70 ist Teil eines Kraftübertragungselementes 72, das an seinem der Zahnleiste 70 abgewandten Ende einen hohlzylindrischen Abschnitt 74 aufweist.

An dem dem Handgriff 62 zugewandten Ende der Auslösestange 66 ist eine Auflage 76 montiert, die zusammen mit einem festen, hohlzylindrischen Anschlag 78 einen Raum definiert, in dem eine auf Druck belastbare Schraubenfeder 80 angeordnet ist.

Die Auslösestange 66 durchsetzt eine in dem Gehäuseteil 56 ausgebildete Durchgangsbohrung 82. Dadurch ist die Auslösestange 66 in Richtung der Längsachse des Gehäuses 52 beweglich geführt. Die Auslösestange 66 dient dazu, eine Entriegelungsmechanik zu betätigen, die in dem in Figur 2 nicht gezeigten Haltearm enthalten ist. Von dieser Entriegelungsmechanik ist in Figur 2 lediglich eine einzelne Entriegelungskomponente 84 dargestellt, die um eine Schwenkachse 86 schwenkbar gelagert ist. Indem die Auslösestange 66 auf die Entriegelungskomponente 84 drückt, wird letztere zur Entriegelung des Haltearms um die Schwenkachse 86 bewegt.

Die Schraubenfeder 80, über die das Kraftübertragungselement 72 mit der Auslösestange 66 gekoppelt ist, bildet einen Kraftbegrenzer, der dafür sorgt, dass die von dem Anwender manuell auf den Handgriff 62 ausgeübte Betätigungskraft über den Umsetzmechanismus 64 nur dann auf die Auslösestange 66 und damit auf die Entriegelungskomponente 84 übertragen wird, wenn die Betätigungskraft eine vorbestimmte Kraft nicht übersteigt.

Die Federkraft, mit der die Schraubenfeder 80 ihrer Kompression entgegenwirkt, ist gerade so gewählt, dass die Schraubenfeder 80 erst dann zusammengedrückt wird, wenn die Betätigungskraft die vorbestimmte Kraft übersteigt. In diesem Fall absorbiert die Schraubenfeder 80 gleichsam die über den Handgriff 62 auf das Kraftübertragungselement 72 ausgeübte Betätigungskraft, indem sie elastisch zusammengedrückt wird. Ist dagegen die Betätigungskraft kleiner oder gleich der vorbestimmten Kraft, so reicht sie nicht aus, die Schraubenfeder 80 zusammenzudrücken. Die Schraubenfeder 80 bildet dann ein starres Kopplungselement zwischen dem Kraftübertragungselement 72 und der Auslösestange 66, so dass die durch Betätigen des Handgriffs 62 verursachte Bewegung des Kraftübertragungselementes 72 in eine entsprechende Bewegung der Auslösestange 66 in Richtung der Längsachse des Gehäuse 52 umgesetzt wird, wodurch die Entriegelungskomponente 84 um die Schwenkachse 86 bewegt und letztlich der Haltearm entriegelt wird.

Die Figuren 2 bis 4 veranschaulichen die Wirkungsweise der Entriegelungsvorrichtung 50, insbesondere des durch die Schraubenfeder 80 gebildeten Kraftbegrenzers.

In Figur 2 ist der Handgriff 62 nicht betätigt. Entsprechend bleibt die Auslösestange 66 in ihrer Verriegelungsstellung, in der sie nicht auf die Entriegelungskomponente 84 wirkt. Der Haltearm ist verriegelt.

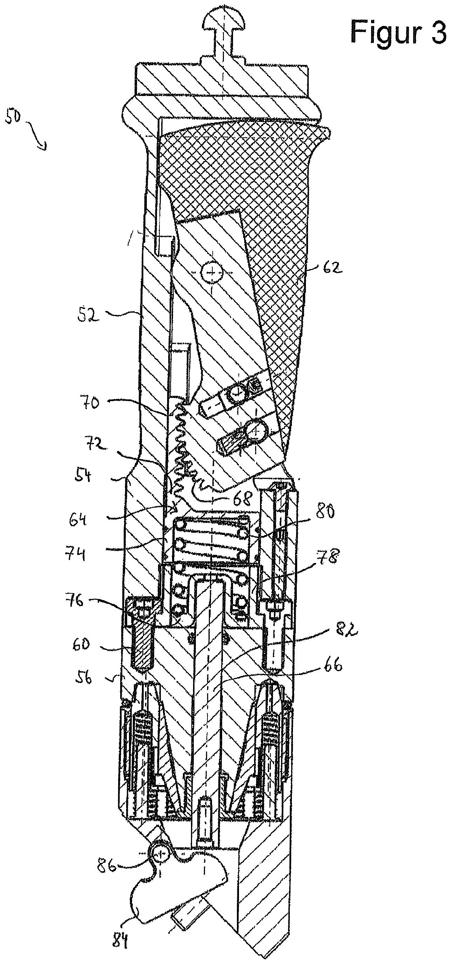

In Figur 3 wird der Handgriff 62 mit einer (lastabhängigen) Betätigungskraft betätigt, die nicht ausreicht, die Schraubenfeder 80 signifikant zusammenzudrücken. In diesem Zustand bildet die Schraubenfeder 80 also ein im Wesentlichen starres Element, das mitsamt dem Kraftübertragungselement 72 in Richtung der Längsachse des Gehäuses 52 nach unten bewegt wird, bis der hohlzylindrische Abschnitt 74 des Kraftübertragungselementes 72 in Anlage mit dem festen Anschlag 78 kommt. Die Betätigungskraft wird also auf die Auslösestange 66 übertragen, wodurch diese in ihre Entriegelungsstellung bewegt wird und die Entriegelungskomponente 84 um die Schwenkachse 86 verschwenkt. Der Haltearm wird entriegelt.

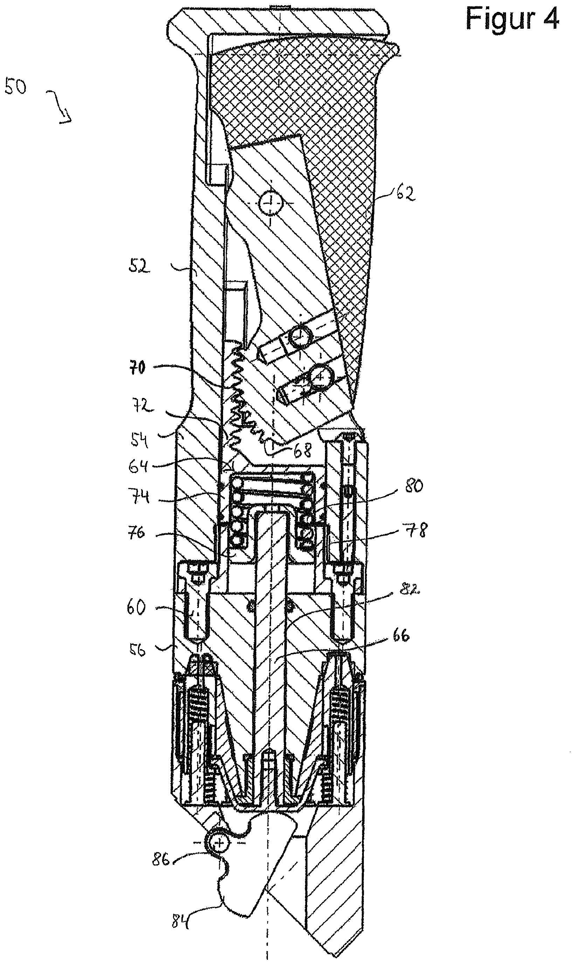

In Figur 4 wird der Handgriff 62 mit einer (lastabhängigen) Betätigungskraft betätigt, die groß genug ist, die Schraubenfeder 80 zusammenzudrücken. Die Schraubenfeder 80 absorbiert so die ausgeübte Betätigungskraft, wodurch die Auslösestange 62 in ihrer Verriegelungsstellung verbleibt und nicht auf die Entriegelungskomponente 84 wirkt. Der Haltearm 62 bleibt trotz Betätigung des Handgriffs 62 verriegelt.

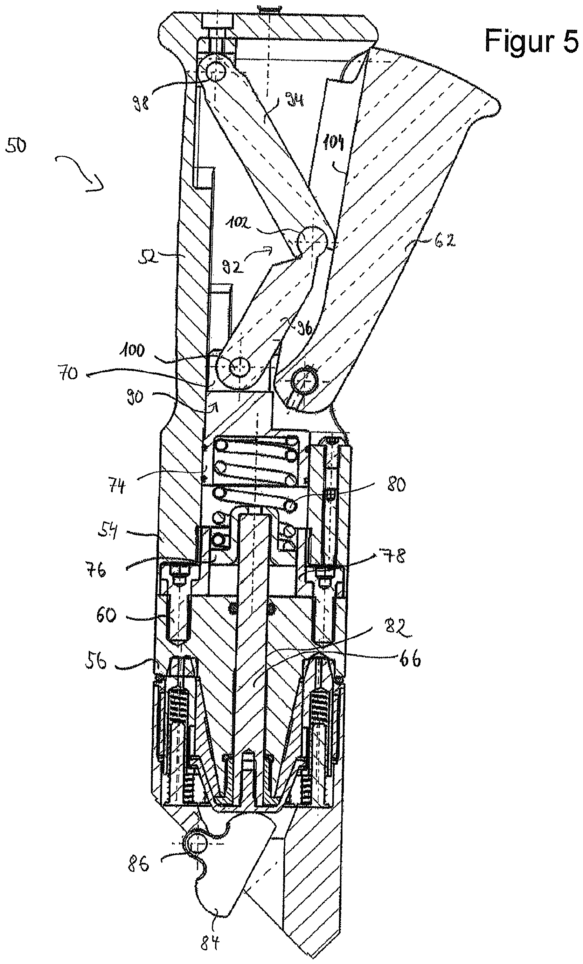

In den Figuren 5 bis 7 ist eine Abwandlung der Entriegelungsvorrichtung 50 als zweites Ausführungsbeispiel dargestellt. Das zweite Ausführungsbeispiel unterscheidet sich von dem in den Figuren 2 bis 4 gezeigten ersten Ausführungsbeispiel lediglich durch einen modifizierten Umsetzmechanismus, der in den Figuren 5 bis 7 mit 90 bezeichnet ist. Diejenigen Komponenten des zweiten Ausführungsbeispiels, die in ihrer Funktion mit denen des ersten Ausführungsbeispiels übereinstimmen, sind mit den in dem ersten Ausführungsbeispiel verwendeten Bezugszeichen versehen.

Der gegenüber dem ersten Ausführungsbeispiel modifizierte Umsetzmechanismus 90 weist einen Kniehebel 92 auf, der aus einem längeren ersten Schenkel 94 und einem kürzeren zweiten Schenkel 96 gebildet ist. Der erste Schenkel 94 ist mit einem Ende an einer Schwenkachse 98 gelagert, die fest in dem Gehäuse 52 montiert ist. Die Schwenkachse 98 bildet so einen relativ zu dem Gehäuse 52 ortsfesten ersten Drehpunkt des Kniehebels 92. Der zweite Schenkel 96 ist mit seinem von dem ersten Schenkel 94 abgewandten Ende an einer Schwenkachse 100 gelagert, die fest an dem Kraftübertragungselement 72 montiert ist, das in Richtung der Längsachse des Gehäuses 52 bewegbar ist. Die Schwenkachse 100 bildet so einen zweiten Drehpunkt, der zusammen mit dem Kraftübertragungselement 72 bewegt wird.

An ihren einander zugewandten Enden sind die beiden Schenkel 94 und 96 drehbar miteinander gekoppelt. Hierzu weist der zweite Schenkel 96 an seinem Ende einen im Querschnitt näherungsweise kugelförmigen Abschnitt 102 auf, der in einer korrespondierenden kugelförmigen Aufnahme gelagert ist, die an dem dem zweiten Schenkel zugewandten Ende des ersten Schenkels 94a ausgebildet ist. Durch diese drehbare Kopplung der beiden Schenkel 94 und 96 ist ein gemeinsamer, beweglicher dritter Drehpunkt des Kniehebels 92 gegeben.

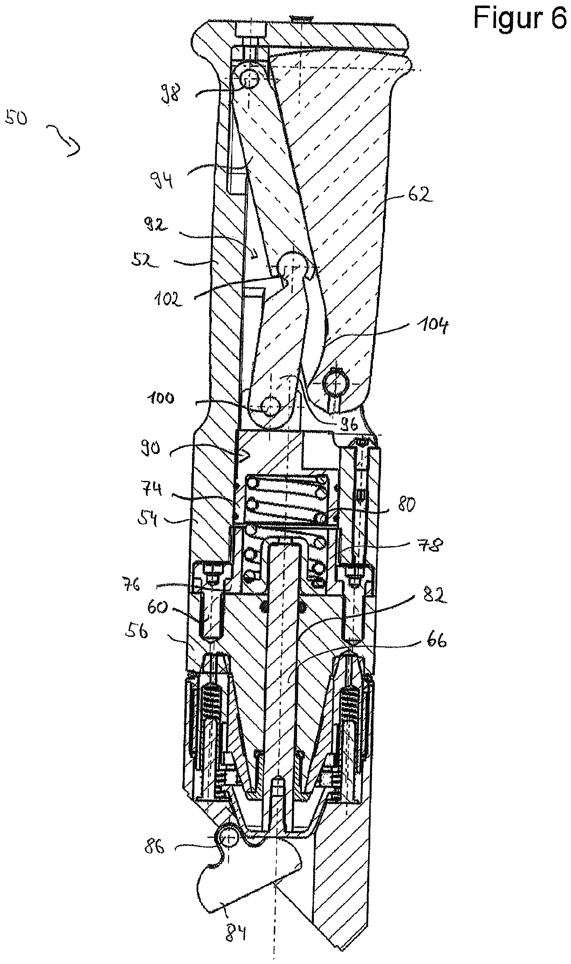

Der Kniehebel 92 liegt im Bereich seines dritten Drehpunktes an einer Andruckfläche 104 an, die an dem Handgriff 62 ausgebildet ist. Betätigt der Anwender den Handgriff 62, so werden die beiden Schenkel 94 und 96, wie in Figur 6 gezeigt, vertikal ausgerichtet, wodurch der Kniehebel 92 insgesamt in eine Form gebracht wird, in der er näherungsweise parallel zur Längsachse des Gehäuses 92 liegt. Durch diese Ausrichtung des Kniehebels 92 wird die auf den Handgriff 62 ausgeübte Betätigungskraft auf das Kraftübertragungselement 72 übertragen, wodurch dieses in Richtung der Längsachse des Gehäuses 52 (in den Figuren 5 bis 7) nach unten bewegt wird.

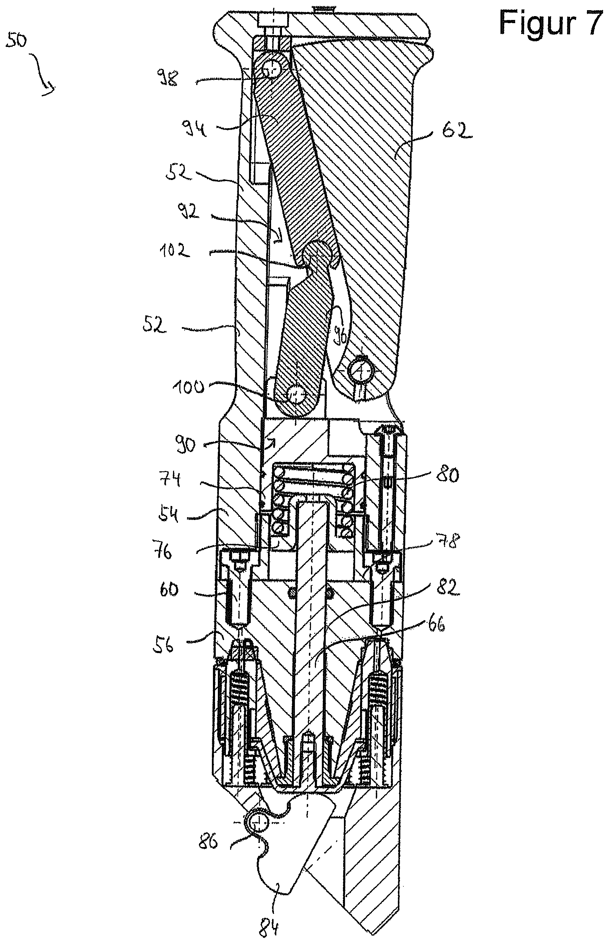

Im Übrigen arbeitet das in den Figuren 5 bis 7 gezeigte zweite Ausführungsbeispiel in gleicher Weise wie das erste Ausführungsbeispiel nach den Figuren 2 bis 4. Dabei entspricht der in Figur 5 gezeigte Zustand, in dem der Handgriff 62 nicht betätigt und dadurch der Haltearm verriegelt ist, dem Zustand nach Figur 2, während der in Figur 6 dargestellte Zustand, in dem der Handgriff 62 betätigt und der Haltearm entriegelt ist, dem Zustand nach Figur 3 entspricht, und der in Figur 7 dargestellte Zustand, in dem der Haltegriff 62 zwar betätigt ist, aber der Haltearm verriegelt bleibt, dem Zustand nach Figur 4 entspricht.

## Patentansprüche

1. Mit einer Last beaufschlagbarer Haltearm (10), der zur Lagerung eines Körperteils eines Patienten ausgebildet ist, umfassend eine Vorrichtung (50) zum manuellen Entriegeln des Haltearms (10), die Vorrichtung (50) umfassend:
ein Bedienteil (62), das zum Entriegeln des Haltearms (10) mit einer Betätigungskraft, die mit zunehmender Last größer wird, manuell zu betätigen ist,
einen Umsetzmechanismus (64, 90), der ein mit dem Bedienteil (62) gekoppeltes Kraftübertragungselement (72) aufweist, und
einen mit dem Kraftübertragungselement (72) gekoppelten Auslöser (66), der zum Entriegeln des Haltearms (10) durch die Betätigungskraft, die das Kraftübertragungselement (72) auf den Auslöser (66) überträgt, aus einer Verriegelungsstellung, in welcher der Auslöser (66) von dem Haltearm wirkungsmäßig entkoppelt ist, in eine Entriegelungsstellung bewegbar ist, in welcher der Auslöser (66) mit dem Haltearm zu dessen Entriegelung wirkungsmäßig gekoppelt ist,
**gekennzeichnet durch** ein längliches Gehäuse (52), in dem der Umsetzmechanismus (64, 90) die auf das Bedienteil (62) manuell ausgeübte Betätigungskraft in eine Bewegung des Kraftübertragungselementes (72) entlang einer Gehäuselängsachse umsetzt,
wobei der Umsetzmechanismus (64, 90) einen entlang der Gehäuselängsachse elastisch verformbaren Kraftbegrenzer (80) aufweist, über den das Kraftübertragungselement (72) mit dem Auslöser (66) gekoppelt ist und der durch seine elastische Verformung die Betätigungskraft auf den Auslöser (66) absorbiert, wenn die Betätigungskraft eine vorbestimmte Kraft übersteigt, wodurch der Auslöser (66) in der Verriegelungsstellung verbleibt.

2. Haltearm (10) nach Anspruch 1, **dadurch gekennzeichnet, dass** der Kraftbegrenzer eine Feder (80) ist, deren Rückstellkraft so bemessen ist, dass die Feder (80) ihre ursprüngliche Form beibehält, wenn die Betätigungskraft kleiner oder gleich der vorbestimmten Kraft ist, und dass die Feder (80) sich elastisch verformt, wenn die Betätigungskraft die vorbestimmte Kraft übersteigt.

3. Haltearm (10) nach Anspruch 2, **dadurch gekennzeichnet, dass** die Feder (80) als Druckfeder ausgebildet ist.

4. Haltearm (10) nach einem der vorhergehenden Ansprüche, **dadurch gekennzeichnet, dass** der Umsetzmetzmechanismus (64) eine an dem Bedienteil (62) ausgebildete erste Zahnung (68) und eine an dem Kraftübertragungselement (72) ausgebildete zweite Zahnung (70) umfasst, die in Eingriff mit der ersten Zahnung (68) steht.

5. Haltearm (10) nach einem der Ansprüche 1 bis 3, **dadurch gekennzeichnet, dass**
der Umsetzmechanismus (90) eine an dem Bedienteil (62) ausgebildete, bewegliche Andruckfläche (104) und einen Kniehebel (92) mit einem längeren ersten Schenkel (94) und einem kürzeren zweiten Schenkel (96) umfasst,
der längere erste Schenkel (94) des Kniehebels (92) an einem Ende in einem innerhalb der Vorrichtung ortsfesten ersten Drehpunkt (92) drehbar gelagert ist,
der kürzere zweite Schenkel (96) des Kniehebels (92) an einem Ende in einem zweiten Drehpunkt (100), der relativ zu dem beweglich geführten Kraftübertragungselement (72) ortsfest ist, drehbar gelagert ist,
die beiden Schenkel (94, 96) jeweils an ihrem anderen Ende in einem gemeinsamen dritten Drehpunkt (100) drehbar gelagert sind, und
die Andruckfläche (104) beim manuellen Betätigen des Bedienteils (62) im Bereich des dritten Drehpunkts (100) auf den Kniehebel (92) drückt.

6. Haltearm (10) nach einem der vorhergehenden Ansprüche, **dadurch gekennzeichnet, dass** das Bedienteil ein schwenkbar gelagerter Handgriff (62) ist.

7. Haltearm (10) nach einem der vorhergehenden Ansprüche, **dadurch gekennzeichnet, dass** das Kraftübertragungselement (72) einen hohlzylindrischen Abschnitt (74) aufweist, in dem der Kraftbegrenzer (80) gelagert ist.

8. Haltearm (10) nach einem der vorhergehenden Ansprüche, **dadurch gekennzeichnet, dass** der Auslöser eine Auslösestange (66) umfasst, die in Richtung ihrer Längsachse linear geführt ist.

9. Haltearm (10) nach Anspruch 8, **dadurch gekennzeichnet**, die Auslösestange (66) zumindest teilweise innerhalb des länglich geformten Kraftbegrenzers (80) geführt ist.

10. Haltearm (10) nach einem der vorhergehenden Ansprüche, **gekennzeichnet durch** einen Anschlag (78), der die elastische Verformung des Kraftbegrenzers (80) begrenzt.

11. Haltearm (10) nach Anspruch 10, **dadurch gekennzeichnet, dass** der Anschlag durch eine mit dem Kraftübertragungselement (72) in Anlage kommende Anschlagfläche gebildet ist.

12. Haltearm (10) nach einem der vorhergehenden Ansprüche, umfassend mindestens zwei starre Halteglieder (12, 14, 16, 18, 20, 22) und ein Gelenk (24, 26, 28, 30, 32), das die beiden Halteglieder (12, 14, 16, 18, 20, 22) miteinander verbindet, und eine Entriegelungsmechanik enthält, die mit dem Auslöser (66) derart zusammenwirkt, dass das Gelenk (24, 26, 28, 30, 32) die beiden Halteglieder (12, 14, 16, 18, 20, 22) in der Verriegelungsstellung des Auslösers (66) starr miteinander koppelt und in der Entriegelungsstellung des Auslösers (66) beweglich miteinander koppelt.

## Claims

1. Support arm, which can be applied with a load (10) and which is configured to position a patient's body part, comprising a device (50) for manual unlocking of the support arm (10), the device (50) comprising:
an operating part (62), which is to be actuated manually by means of an actuation force, which increases with increasing load, to unlock the support arm (10),
a transformation mechanism (64, 90), having a power transmission element (72), which is coupled to the operating part (62), and
an actuator (66) being coupled to the power transmission element (72), the actuator being movable to unlock the support arm (10) by means of the actuation force, which transmits the power transmission element (72) to the actuator (66) from a locked position, in which the actuator (66) is operatively decoupled from the support arm, into an unlocked position, in which the actuator (66) is operatively coupled to the support arm for unlocking the same,
**characterized by** an elongated housing (52), in which the transmission mechanism (64, 90) transmits the manually exerted actuation force applied to the operating part (62) into a movement of the power transmission element (72) along a longitudinal axis of the housing,
the transformation mechanism (64, 90) having an elastically deformable force limiter (80) along the longitudinal axis of the housing, by means of which the power transmission element (72) is coupled to the actuator (66) and which absorbs the actuation force to the actuator (66) by means of its elastic deformation, if the actuation force exceeds a predetermined force, which causes the actuator (66) to remain in the locked position.

2. Support arm (10) according to claim 1, **characterized in that** the force limiter is a spring (80), the reset force of which being designed such that the spring (80) maintains its original shape if the actuation force is less than or equal to the predetermined force and that the spring (80) elastically deforms if the actuation force exceeds the predetermined force.

3. Support arm (10) according to claim 2, **characterized in that** the spring (80) is formed as a pressure spring.

4. Support arm (10) according to any of the preceding claims, **characterized in that** the transformation mechanism (64) comprises a first toothing (68) formed at the operating part (62), and a second toothing (70) formed at the power transmission element (72), which second toothing engages with the first toothing (68).

5. Support arm (10) according to any of the claims 1 to 3, **characterized in that**
the transformation mechanism (90) comprises a movable press-on surface (104) formed at the operating part (62), and a knee lever (92) having one longer first leg (94) and one shorter second leg (96),
the longer first leg (94) of the knee lever (92) being pivotally mounted at one end within a first rotation point (92), which is stationary within the device,
the shorter second leg (96) of the knee lever (92) being pivotably mounted at one end within a second rotation point (100), which is stationary with respect to the movably guided power transmission element (72),
the two legs (94, 96) being pivotably mounted in a common third rotation point (100) at their respective other ends; and
the press-on surface (104) pressing on the knee lever (92) when manually actuating the operating part (62) within the area of the third rotation point (100).

6. Support arm (10) according to any of the preceding claims, **characterized in that** the operating part is a pivotably mounted handle (62).

7. Support arm (10) according to any of the preceding claims, **characterized in that** the power transmission element (72) has a hollow cylindrical portion (74), in which the force limiter (80) is mounted.

8. Support arm (10) according to any of the preceding claims, **characterized in that** the actuator comprises an actuation rod (66), which is guided linearly in the direction of its longitudinal axis.

9. Support arm (10) according to claim 8, **characterized by** the actuation rod (66) being guided at least partially within the longitudinally shaped force limiter (80).

10. Support arm (10) according to any of the preceding claims, **characterized by** a stop (78), which limits the elastic deforming of the force limiter (80).

11. Support arm (10) according to claim 10, **characterized in that** the stop is formed by a stop surface, which comes into contact with the power transmission element (72).

12. Support arm (10) according to any of the preceding claims, comprising at least two rigid support elements (12, 14, 16, 18, 20, 22) and a joint (24, 26, 28, 30, 32), which connects the two support elements (12, 14, 16, 18, 20, 22) to one another, and including unlocking mechanics, which cooperates with the actuator (66) such that the joint (24, 26, 28, 30, 32) rigidly couples the two support elements (12, 14, 16, 18, 20, 22) to one another in the locked position of the actuator (66), and couples them movably to one another in the unlocked position of the actuator (66).

## Revendications

1. Bras de maintien (10) pouvant être soumis à l'action d'une charge, lequel est réalisé afin d'accueillir une partie corporelle d'un patient, comprenant un dispositif (50) servant à déverrouiller manuellement le bras de maintien (10), lequel dispositif (50) comprend :
une partie de commande (62), qui est à actionner manuellement afin de déverrouiller le bras de maintien (10) avec une force d'actionnement, qui devient plus grande au fur et à mesure que la charge augmente,
un mécanisme de conversion (64, 90), qui présente un élément de transmission de force (72) couplé à la partie de commande (62), et
un déclencheur (66) couplé à l'élément de transmission de force (72), qui peut être déplacé, aux fins du déverrouillage du bras de maintien (10), par la force d'actionnement, que l'élément de transmission de force (72) transmet au déclencheur (66), d'une position de verrouillage, dans laquelle le déclencheur (66) est découplé d'un point de vue fonctionnel du bras de maintien, à une position de déverrouillage, dans laquelle le déclencheur (66) est couplé d'un point de vue fonctionnel au bras de maintien aux fins du déverrouillage de ce dernier,
**caractérisé par** un boîtier (52) allongé, dans lequel le mécanisme de conversion (64, 90) convertit la force d'actionnement exercée manuellement sur la partie de commande (62) en un déplacement de l'élément de transmission de force (72) le long d'un axe longitudinal du boîtier,
dans lequel le mécanisme de conversion (64, 90) présente un limiteur de force (80) pouvant être déformé élastiquement le long de l'axe longitudinal du boîtier, par l'intermédiaire duquel l'élément de transmission de force (72) est couplé au déclencheur (66) et qui absorbe du fait de sa déformation élastique la force d'actionnement sur le déclencheur (66) quand la force d'actionnement dépasse une force prédéfinie, moyennant quoi le déclencheur (66) reste dans la position de verrouillage.

2. Bras de maintien (10) selon la revendication 1, **caractérisé en ce que** le limiteur de force est un ressort (80), dont la force de rappel présente des valeurs telles que le ressort (80) conserve sa forme d'origine quand la force d'actionnement est inférieure ou égale à la force prédéfinie, et que le ressort (80) se déforme élastiquement quand la force d'actionnement dépasse la force prédéfinie.

3. Bras de maintien (10) selon la revendication 2, **caractérisé en ce que** le ressort (80) est réalisé sous la forme d'un ressort de pression.

4. Bras de maintien (10) selon l'une quelconque des revendications précédentes, **caractérisé en ce que** le mécanisme de conversion (64) comprend une première denture (68) réalisée au niveau de la partie de commande (62) et une deuxième denture (70) réalisée au niveau de l'élément de transmission de force (72), laquelle est en prise avec la première denture (68).

5. Bras de maintien (10) selon l'une quelconque des revendications 1 à 3, **caractérisé en ce que**
le mécanisme de conversion (90) comprend une surface de compression (104) mobile réalisée au niveau de la partie de commande (62) et un levier coudé (92) pourvu d'une première branche (94) plus longue et d'une deuxième branche (96) plus courte,
la première branche (94) plus longue du levier coudé (92) est montée de manière à pouvoir pivoter au niveau d'une extrémité en un premier point de rotation (92) stationnaire à l'intérieur du dispositif,
la deuxième branche (96) plus courte du levier coudé (92) est montée de manière à pouvoir pivoter au niveau d'une extrémité en un second point de rotation (100), qui est stationnaire par rapport à l'élément de transmission de force (72) guidé de manière mobile,
les deux branches (94, 96) sont chacune montées de manière à pouvoir pivoter au niveau de leur autre extrémité en un troisième point de rotation (100) commun, et
**en ce que** la surface de compression (104) exerce une pression sur le levier coudé (92) lors de l'actionnement manuel de la partie de commande (62) dans la zone du troisième point de rotation (100).

6. Bras de maintien (10) selon l'une quelconque des revendications précédentes, **caractérisé en ce que** la partie de commande est une poignée (62) montée de manière à pouvoir s'incliner.

7. Bras de maintien (10) selon l'une quelconque des revendications précédentes, **caractérisé en ce que** l'élément de transmission de force (72) présente une section (74) cylindrique creuse, dans laquelle le limiteur de force (80) est monté.

8. Bras de maintien (10) selon l'une quelconque des revendications précédentes, **caractérisé en ce que** le déclencheur comprend une tige de déclenchement (66), qui est guidée de manière linéaire dans le sens de son axe longitudinal.

9. Bras de maintien (10) selon la revendication 8, **caractérisé en ce que** la tige de déclenchement (66) est guidée au moins en partie à l'intérieur du limiteur de force (80) de forme allongée.

10. Bras de maintien (10) selon l'une quelconque des revendications précédentes, **caractérisé par** une butée (78), qui limite la déformation élastique du limiteur de force (80).

11. Bras de maintien (10) selon la revendication 10, **caractérisé en ce que** la butée est formée par une surface de butée venant en appui avec l'élément de transmission de force (72).

12. Bras de maintien (10) selon l'une quelconque des revendications précédentes, comprenant au moins deux organes de maintien (12, 14, 16, 18, 20, 22) rigides et une articulation (24, 26, 28, 30, 32), qui relie l'un à l'autre les deux organes de maintien (12, 14, 16, 18, 20, 22), et contient un système mécanique de déverrouillage, qui interagit avec le déclencheur (66) de telle manière que l'articulation (24, 26, 28, 30, 32) couple l'un à l'autre de manière rigide les deux organes de maintien (12, 14, 16, 18, 20, 22) dans la position de verrouillage du déclencheur (66) et les couple de manière mobile l'un à l'autre dans la position de déverrouillage du déclencheur (66).
